# FASCICULE DE BREVET EUROPEEN

(11) **EP 1 032 401 B1**
(45) Date de publication et mention de la délivrance du brevet: **16.03.2005**
(21) Numéro de dépôt: 98956724.3
(22) Date de dépôt: 25.11.1998
(51) Int. Cl.: A61K 31/655, A61P 37/00

(54) **PROCEDE D'INHIBITION DE LA PRODUCTION CELLULAIRE DE CYTOKINES**
VERFAHREN ZUR HEMMUNG DER ZELLULÄREN ZYTOKINPRODUKTION
METHOD FOR INHIBITING CYTOKINE PRODUCTION BY CELLS

(30) Priorité: 26.11.1997 BE 9700949
(43) Date de publication de la demande: 06.09.2000
(73) Titulaire: Previsan AG, 6301 Zug (CH)
(72) Inventeur: GOLDMAN, Michel, B-1180 Bruxelles (BE); MARGERY, Hélène, B-1301 Bièrges (BE); ROBBERECHT, Patrick, Adelin, Oscar, B-1170 Bruxelles (BE); TASSIGNON, Jean, Pierre, Robert, Ghislain, B-1170 Bruxelles (BE); VANDEVELDE, Michel, B-1000 Bruxelles (BE)
(74) Mandataire: Claeys, Pierre
(86) Numéro de dépôt international: PCT/BE1998/000183
(87) Numéro de publication internationale: WO 1999/027937

(56) Documents cités:
- WO-A-91/16054
- O.A. LUK'YANOV ET AL. : "N'-(alpha-Acetoxyiminoalkyl)diazene N-oxides and some of their transformations" IZV. AKAD. NAUK. SSR, SER. KHIM., no. 1, 1991, pages 109-114, XP002100163
- Y MAKINO ET AL: "Thioredoxin: a redox-regulating cellular cofactor for glucocorticoid hormone action. Cross talk between endocrine control of stress response and cellular antioxidant defense system" DIALOG CANCERLIT, vol. 11, no. 98, 1 janvier 1996 (1996-01-01), page 2469 2477 XP002073578
- J C NORMAND ET AL: "Occupational asthma after exposure to azodicarbonamide: report of four cases" DIALOG MEDLINE, vol. 1, no. 46, 1 janvier 1989 (1989-01-01), page 60 62 XP002073577
- V M YATES ET AL: "Contact dermatitis from azodicarbonamide in earplugs" DIALOG MEDLINE, vol. 2, no. 19, 1 août 1988 (1988-08-01), page 155 156 XP002073576
- A K SAMANTA ET AL: "Modification of sulfhydryl groups of interleukin-8 (IL-8) receptor impairs binding of IL-8 and IL-8-mediated chemotactic response of human polymorphonuclear neutrophils" DIALOG MEDLINE, vol. 9, no. 268, 25 mars 1993 (1993-03-25), page 6147 6153 XP002073575
- P BRENNAN ET AL: "The effects of thiol modifiers on the activation of NF kappa B by interleukin-1" DIALOG MEDLINE, vol. 4, no. 21, 1 novembre 1993 (1993-11-01), page 390S XP002073574
- P BRENNAN ET AL: "Inhibition of NF kappa B activity by oxidative processes in intact cells mechanism of action of pyrolidine dithiocarbamate and diamide" DIALOG MEDLINE, vol. 1, no. 24, 1 février 1996 (1996-02-01), page 3S XP002073573
- C MENDEZ ET AL: "Oxidants augment endotoxin-induced activation of alveolar macrophages" DIALOG MEDLINE, vol. 3, no. 6, 1 septembre 1996 (1996-09-01), page 157 163 XP002073572
- P MARCHETTI ET AL: "Redox regulation of apoptosis: impact of thiol oxidation status on mitochondrial function" DIALOG MEDLINE, vol. 1, no. 27, 1 janvier 1997 (1997-01-01), page 289 296 XP002073571
- K KINOSHITA ET AL: "Reducing environment protects sinusoidal lymphocytes isolated from normal human liver from apoptosis" DIALOG MEDLINE, vol. 1, no. 26, 1 janvier 1997 (1997-01-01), page 103 110 XP002073570
- R W WATSON ET AL: "Thiol-mediated redox regulation of neutrophil apoptosis" DIALOG MEDLINE, vol. 2, no. 120, 1 août 1996 (1996-08-01), page 150 157 XP002073569
- S DHAWAN ET AL: "Induction of endothelial cell surface adhesion molecules by tumor necrosis factor is blocked by protein tyrosine phosphatase inhibitors: role of the nuclear transcription factor NF-kappa B" DIALOG MEDLINE, vol. 9, no. 27, 1 septembre 1997 (1997-09-01), page 2172 2179 XP002073568
- N SATO ET AL: "Thiol-mediated redox regulation of apoptosis. Possible roles of cellular thiols other than glutathione in T cell apoptosis" DIALOG CANCERLIT, vol. 7, no. 154, 1 janvier 1995 (1995-01-01), page 3194 3203 XP002073567
- S SINGH ET AL: "Protein-tyrosine phosphatase inhibitors block tumor necrosis factor-dependent activation of the nuclear transcription factor NF -kappa B" DIALOG CANCERLIT, vol. 18, no. 270, 1 janvier 1995 (1995-01-01), page 10631 10639 XP002073566

## Description

La présente invention est relative à un procédé d'inhibition in vitro de production de cytokines par des cellules, notamment animales ou humaines, et de leur sécrétion.

La présente invention a pour but de mettre au point un procédé d'inhibition in vitro de production de cytokines par des cellules qui ne mette pas en danger ces cellules. Avantageusement, ce procédé permettra d'inhiber chez ces cellules la production et la sécrétion de substances favorisant l'apparition de phénomènes immunoallergiques.

On résout ce problème suivant l'invention par un procédé tel que décrit au début, comprenant une application sur lesdites cellules d'au moins un des dérivés azoïques répondant à la formule 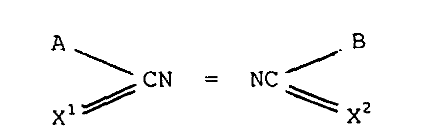 dans laquelle A représente un groupe carboxyle ou un groupe 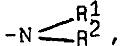 B représente un groupe carboxyle ou un groupe 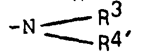 R¹, R², R³ et R⁴ sont identiques ou différents et représentent chacun indépendamment un atome d'hydrogène ou d'halogène, un radical d'hydrocarbure aliphatique ou aromatique, éventuellement substitué, ou un groupe hydroxy, R¹ et R² pouvant être liés ensemble pour former un noyau hétérocyclique avec leur atome d'azote adjacent, et R³ et R⁴ pouvant être liés ensemble pour former un noyau hétérocyclique avec leur atome d'azote adjacent, X¹ et X² sont identiques ou différents et représentent chacun indépendamment un atome d'oxygène ou un groupe NR⁵, dans lequel R⁵ est un atome d'hydrogène ou d'halogène, un radical d'hydrocarbure aliphatique ou aromatique, éventuellement substitué, ou un groupe nitro, et dans lequel, lorsque deux groupes NR⁵ sont simultanément présents, chaque R⁵ peut être identique à ou différent de l'autre, ainsi que de leurs sels, esters et isomères.

Plusieurs de ces dérivés azoïques sont des composés connus, en particulier pour leur activité antivirale, notamment contre les virus du groupe rétrovirus, en particulier le virus du SIDA (voir WO-A-9116054 et WO-A-9107876, ainsi que le US-A-5585367).

En ce qui concerne plus particulièrement le 1,1'-azobisdiméthylformamide, appelé aussi diamide, de nombreux chercheurs se sont penchés sur le phénomène d'activation du facteur de transcription. intracellulaire NF kappa B et ont montré que le diamide bloque le rôle de certaines enzymes dans la cascade d'activation de ce facteur (S. Singh et al., Protein-tyrosine..., The Journal of Biological Chemistry, 270(18), 1995; Brennan et al., Inhibition of NFkappa B..., Biochemical Society Transactions 24(1), 1996; Brennan et al, The effects of thiol..., Biochemical Society Trans. 21(4), 1993).

D'autres auteurs ont montré qu'à certaines concentrations le diamide induit une apoptose dans certaines lignées cellulaires.

D'autres auteurs encore ont montré un effet du diamide sur certains récepteurs membranaires.

Aucune de ces études ne montre cependant une action d'inhibition du diamide sur la production de cytokines par les cellules observées. On peut même noter que C. Mendez et al, dans Oxidants augment endotoxin - induced activation of alveolar macrophages, SHOGK, vol. 6, n° 3, p. 157-163, 1996, observe une augmentation non significative de la production du facteur de nécrose tumorale à la dose de 1 millimole, c'est-à-dire un effet inverse à celui observé de manière répétitive sur diverses cytokines suivant la présente invention.

Il est par ailleurs connu que les dérivés azoïques suivant l'invention offrent une toxicité propre extrêmement faible en soi vis-à-vis du corps humain ou des cellules saines traitées.

Suivant un mode de réalisation de l'invention, le procédé comprend une inhibition in vitro de production et de sécrétion d'interleukines par les cellules. Parmi le groupe des interleukines on peut citer notamment les interleukines IL-2, IL-4, IL-5, IL-6, IL-8, IL-10, IL-12 et IL-15, et on envisage tout particulièrement l'inhibition d'IL-2 et d'IL-5.

Suivant un autre mode de réalisation de l'invention, le procédé comprend une inhibition de production et de sécrétion d'interféron-γ (IFN-γ) par les cellules. Suivant encore un autre mode de réalisation, le procédé comprend une inhibition de production et de sécrétion d'un facteur de nécrose tumorale α (TNF-α).

Suivant un mode de réalisation avantageux de l'invention, R₁ à R₅ représentent chacun dans la formule générale donnée ci-dessus un radical d'hydrocarbure aliphatique ou aromatique comportant de 1 à 6 atomes de carbone. Les hétérocycles éventuellement formés dans la formule générale peuvent contenir, outre un atome d'azote, au moins un autre hétéroatome, par exemple de l'oxygène. L'hétérocycle est par exemple pentagonal à octogonal, de préférence il est hexagonal. Le dérivé azoïque suivant l'invention peut être choisi parmi le groupe comprenant des dérivés d'azobisformamidine, tels que de la 1,1'-azobisformamidine, de la 1,1-azobisnitroformamidine, de la 2,2'-azobisméthylformamidine, de la 1,1'-azobisfluoroformamidine, de la 1-monochloroazobisformamidine, et de la azobis-[chloroformamidine], des dérivés d'azobisformamide, tels que du 1,1'-azobisformamide et du diméthyl-azobisformamide, de la 1,1'-(azodicarbonyl)-dipipéridine, de la 1,1'-(azodicarbonyl)-dimorpholine, de l'acide azodihydroxamique et ses sels, et de l'acide azodicarboxylique et ses sels.

Avantageusement le procédé comprend une application du dérivé azoïque sur les cellules à une dose n'induisant pas l'apoptose de celles-ci. On peut de préférence envisager une concentration micromolaire d'environ 0,4 à environ 200, de préférence de 2 à 20, avantageusement de 2 à 10.

Sous cet aspect, le 1,1'-azobisdiméthylformamide, appelé aussi diamide, peut présenter dans certains circonstances l'inconvénient de provoquer à certaines concentrations trop élevées une apoptose cellulaire, tout en offrant une demi-vie relativement courte, de seulement quelques minutes.

Suivant une forme de réalisation de l'invention, l'application d'au moins un des dérivés azoïques indiqués est effectuée sur des cellules isolées de macroorganismes ou des cellules de microorganismes, qui par exemple proviennent de cultures cellulaires. Les cellules traitées peuvent aussi être celles d'un organe ou tissu multicellulaire extrait d'un corps humain ou animal, comme par exemple des cellules d'un échantillon de sang ou de lymphe.

Suivant une forme de réalisation de l'invention, l'application d'au moins un des dérivés azoïques indiqués est par exemple effectuée sur des cellules isolées de sang humain productrices de cytokines. Ces cellules peuvent être extraites de manière globale et dès lors on observera des effets globaux sur les différents types lymphocytaires, des cellules présentatrices d'antigènes ainsi que sur des macrophages en cocultures. Une étape plus poussée de l'investigation pourra consister en la mesure de la production des cytokines par des lignées cellulaires hautement purifiées, par exemple des lymphocytes CD4.

Le traitement des lignées cellulaires concernées peut aussi se faire par l'administration des dérivés azoïques à des organismes vivants en ce compris l'être humain et la mesure des différents groupes de cytokines dans les liquides biologiques extraits du corps humain ou animal, avant, pendant et/ou après traitement, ainsi que par la mesure de la possibilité de production des lymphokines concernées après extraction des cellules contenues dans ces liquides.

On peut aussi envisager un traitement par un dérivé azoïque suivant l'invention de greffons ou de tissus cellulaires avant leur transplantation dans un corps humain ou animal.

On peut aussi prévoir l'utilisation de médicaments à base des dérivés azoïques suivant l'invention pour traiter des patients contre le rejet de greffes.

Les cytokines sont produites dans l'organisme par différents réservoirs cellulaires.

Il est connu par exemple que les lymphocytes se spécialisent pour la production spécifique de cytokines et que, selon le type de cytokines produites, on différencie des lymphocytes CD4 Th1 et Th2.

Des cytokines sont aussi produites par les lymphocytes CD8 (IL-4 et IL-5) mais aussi par les mastocytes et les éosinophiles et au stade d'implantation dans l'utérus par les cellules ectodermiques du trophoblaste.

Ces productions de cytokines à partir de ces différents réservoirs cellulaires interviennent dans l'apparition de divers processus inflammatoires et allergiques au sens large, ainsi que dans le phénomène de rejets de greffes. On peut citer entre autres de manière non. exhaustive les pathologies suivantes : l'asthme, la dermatite atopique, la rhinite allergique saisonnière, le psoriasis, le pemphigus, la thyroïdite, la myasthénie, la polyarthrite rhumatoïde, la néphropathie à Ig A, la sclérodermie, le lupus érythémateux, le diabète insulino-dépendant, la sclérose en plaque, les maladies inflammatoires du tube digestif, la maladie de Crohn, l'hyperéosinophilie, le syndrome éosinophile, ainsi que toute affection due à une apoptose cellulaire médiatisée par IL-5.

La substance active suivant l'invention peut être appliquée sur les cellules, seule ou en mélange avec d'autres substances suivant l'invention, ou encore en mélange avec d'autres substances ayant un autre effet sur les cellules. On peut prévoir l'application de la ou des substances actives telles quelles ou sous la forme d'une composition comportant au moins un des dérivés azoïques répondant à la formule donnée précédemment et un support ou véhicule approprié.

Certains composés suivant l'invention présentent au moins un atome de C asymétrique et par conséquent tous les isomères, y compris les diastéréoisomères et les isomères rotatifs ou énantiomères, sont englobés comme faisant partie de l'invention. L'invention inclut les isomères D et L sous forme pure ou en mélange, y compris les mélanges racémiques.

Certains composés de type acide par exemple les acides carboxyliques peuvent former des sels par exemple avec des métaux, ou avec des amines acceptables, ou des esters avec des alcools compatibles.

La présente invention est aussi relative à l'utilisation d'au moins un des dérivés répondant à la formule donnée précédemment, ainsi que de leurs sels ou isomères, pour la fabrication de médicaments à mettre en oeuvre dans le traitement ou la prophylaxie d'affections humaines ou animales résultant d'une production cellulaire pathologique desdites cytokines. On peut spécialement prévoir la fabrication de médicaments pour le traitement et/ou la prévention de maladies auto-immunes, et/ou inflammatoires faisant intervenir les lymphocytes T, de maladies inflammatoires allergiques ou encore du rejet d'allogreffe et/ou de xénogreffe d'organes et de la maladie du greffon contre l'hôte après allogreffe cellulaire. Avantageusement ladite utilisation sera donc prévue pour fabriquer des médicaments destinés à être mis en oeuvre dans le traitement ou la prophylaxie d'affections telles que celles indiquées précédemment.

Le médicament ainsi préparé suivant l'invention peut être administré par toute voie appropriée, entre autres par voie orale, sublinguale, rectale ou vaginale, par injection ou perfusion, par voie locale, transcutanée ou transmuqueuse. Le médicament contient une quantité thérapeutiquement efficace du ou des dérivés azoïques indiqués précédemment. Le dosage sera variable d'individu à individu en fonction de leurs propres caractéristiques immunologiques qui sont en partie génétiquement déterminées. Le médicament peut se présenter sous n'importe quelle forme galénique appropriée, par exemple sous la forme de gélules, de pilules, de comprimés, de dragées, de poudres, de formes à injecter, de crèmes, d'onguents, de systèmes connus de distribution transdermique, de produits à inhaler.

Les formulations et compositions pharmaceutiques peuvent contenir des excipients pharmaceutiquement acceptables usuels, ainsi qu'éventuellement des additifs courants en pharmacie. Ces excipients et additifs comprennent notamment des charges inertes compatibles, des liants, des agents de désintégration, des tampons, des agents conservateurs, des anti-oxydants, des lubrifiants, des agents sapides, des épaississants, des colorants, des émulsionnants, etc...

Etant donné leur première application dans le domaine thérapeutique, un objet de l'invention concerne également des dérivés azoïques répondant à la formule générale donnée précédemment, dans laquelle au moins un des groupes A et B représente un groupe carboxyle, ainsi que leurs sels, esters et isomères pharmaceutiquement acceptables, pour leur application en tant que substances thérapeutiquement actives. On envisage en particulier, comme dérivés de ce genre, des acides azodicarboxyliques de la formule ci-dessous 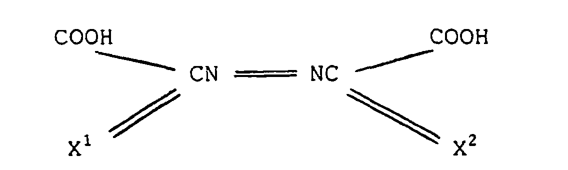 et leurs sels, esters et isomères.

De même, un autre objet de l'invention concerne des dérivés azoïques répondant à la formule générale donnée précédemment, dans laquelle le groupe A représente le groupe 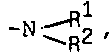 l'un des radicaux R¹ et R² représentant un groupe hydroxy et l'autre un atome d'hydrogène, et dans laquelle le groupe B peut représenter simultanément le groupe 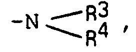 l'un des radicaux R³ et R⁴ pouvant représenter un groupe hydroxy et l'autre un atome d'hydrogène, ainsi que leurs sels, esters, et isomères pharmaceutiquement acceptables, pour leur application en tant que substances thérapeutiquement actives. On envisage en particulier . l'acide azodihydroxamique de la formule ci-dessous 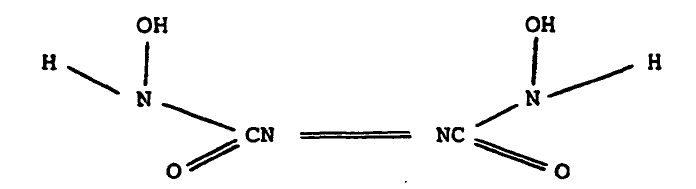 et ses sels et isomères.

De même enfin, un autre objet de l'invention concerne la 1,1'-(azodicarbonyl)-dimorpholine de formule 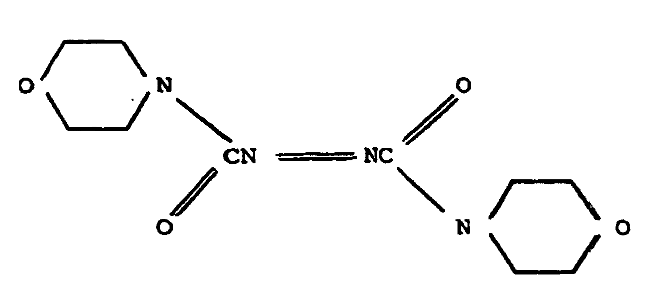 ainsi que ses sels, esters et isomères, pour leur application en tant que substances thérapeutiquement actives.

Toutes ces substances présentant pour la première fois une application dans le domaine thérapeutique sont destinées en particulier à leur mise en oeuvre dans le traitement ou la prophylaxie d'affections humaines ou animales résultant d'une production cellulaire pathologique de cytokines.

L'invention concerne également des produits contenant au moins un des dérivés azoïques répondant à la formule indiquée précédemment et de leurs isomères et au moins une cytokine, comme produit de combinaison pour une utilisation simultanée, séparée ou étalée dans le temps, dans la thérapie ou la prophylaxie d'affections humaines ou animales résultant d'une production cellulaire pathologique d'au moins une cytokine, différente de ladite au moins une cytokine contenue dans le produit de combinaison, ou dans la thérapie ou la prophylaxie d'affections humaines ayant pour effet secondaire une production cellulaire pathologique d'au moins une cytokine, différente de ladite au moins une cytokine contenue dans le produit de combinaison.

D'autres formes et modes de réalisation de l'invention sont indiqués aux revendications annexées.

L'invention va à présent être expliquée de manière plus détaillée à l'aide des exemples donnés ci-dessous à titre illustratif, et non limitatif.

Les expériences décrites dans ces exemples ont été effectuées au Laboratoire d'Immunologie Expérimentale de l'Université Libre de Bruxelles, à l'exception de l'expérience de l'exemple 7, qui a été réalisée dans les laboratoires de la firme UCB S.A.

### Exemple 1

### Effet de l'ADA sur la production de IL-5.

Des cellules mononucléaires de sang périphérique (PMBC) de 5 donneurs humains sains sont isolées d'une manière courante à partir de 50 ml de leur sang et sont ensuite purifiées par centrifugation sur gradient de densité (Lymphoprep). Les cellules (5.10⁶ cellules/ml) sont activées par 0,3 µg/ml de phytohémagglutinine (PHA) et mises en culture pendant 72 heures, à 37°C, en présence de diverses concentrations en ADA (20, 10, 5, 1, 0,2 et 0,04 µg/ml). Le milieu de culture consiste en RPMI 1640 complété de 10% de sérum de veau foetal et de glutamine, et il contient du mercaptoéthanol. Les solutions d'ADA sont préparées dans du diméthylsulfoxyde (DMSO), la concentration de travail en DMSO pendant les expériences étant de 0,1%. Ces dernières sont réalisées sur des plaques à 96 puits (200 µl/puits) et après centrifugation des plaques à 1500 tours par minute pendant 10 minutes, les surnageants sont récoltés et analysés pour leur teneur en IL-5 par un dosage immuno-enzymatique ELISA.

Les résultats de cette expérience ressortent du tableau 1 donné ci-dessous.

**Tableau 1**

| Teneur en IL-5 en pourcentage de l'essai témoin | | | | | | | |
|---|---|---|---|---|---|---|---|
| Concentration en ADA (µg/ml) | 0 | 0,04 | 0,2 | 1 | 5 | 10 | 20 |
| Donneur 1 | 100 | 83 | 54 | 48 | 44 | 29 | 9 |
| Donneur 2 | 100 | 50 | 42 | 29 | 2 | 2 | 2 |
| Donneur 3 | 100 | 58 | 63 | 57 | 23 | 28 | 26 |
| Donneur 4 | 100 | 46 | 57 | 37 | 31 | 14 | 5 |
| Donneur 5 | 100 | 55 | 58 | 49 | 34 | 31 | 7 |
| Moyenne | 100 | 58 | 55 | 44 | 27 | 21 | 10 |

Ces résultats montrent que, comparativement aux cellules non traitées, les PBMC des 5 donneurs testés produisent moins d'IL-5 en présence d'ADA. En moyenne, la production d'IL-5 est inhibée à 90% pour 20 µg/ml d'ADA, à 80% pour 10 µg/ml et à 45% pour 0,2 µg/ml.

Il faut noter que c'est la première fois qu'un effet de l'ADA est mis en évidence d'une manière tout à fait surprenante à des concentrations aussi faibles.

Des essais comparables ont été effectués en l'absence de mercaptoéthanol avec des résultats relativement semblables.

### Exemple 2

### Effet de l'ADA sur la production d'interféron-γ.

A partir des mêmes cultures cellulaires que celles utilisées dans l'Exemple 1, on a examiné la présence d'interféron γ après traitement aux concentrations indiquées d'ADA. Ici aussi, pour 4 donneurs sur 5, on observe une inhibition, ainsi qu'il ressort du tableau 2 ci-dessous.

**Tableau 2**

| Teneur en interféron-γ en pourcentage de l'essai témoin | | | | | | | |
|---|---|---|---|---|---|---|---|
| Concentration en ADA (µg/ml) | 0 | 0,04 | 0,2 | 1 | 5 | 10 | 20 |
| Donneur 1 | 100 | 62 | 112 | 87 | 68 | 23 | 21 |
| Donneur 2 | 100 | 53 | 111 | 98 | 128 | 169 | 86 |
| Donneur 3 | 100 | 99 | 70 | 49 | 62 | 43 | 40 |
| Donneur 4 | 100 | 82 | - | - | 57 | - | 25 |
| Donneur 5 | 100 | 38 | - | - | 25 | - | 16 |
| Moyenne | 100 | 67 | 98 | 78 | 68 | 78 | 38 |

Dans ces deux exemples la diminution de production des cytokines dépend de la quantité d'ADA mise en oeuvre (effet dépendant de la dose) et elle est observée pour des concentrations en ADA n'induisant pas d'effets cytotoxiques.

### Exemple 3

### Effet de l'ADA sur la production d'IL-2, d'IL-5 et d'interféron-γ par des lymphocytes T purifiés.

Des PBMC de 3 donneurs sains sont purifiées par centrifugation sur gradient de densité (LymphoPrep). Les lymphocytes T sont obtenus à partir de 15.10⁶ PMBC incubées pendant 20 à 30 minutes dans un bain-marie de 37°C avec 0,8 ml d'un mélange LymphoKwik® (One Lambda, Inc., CA, USA). Celui-ci est un mélange de complément et d'anticorps spécifique pour des motifs antigéniques de la membrane des cellules qui lysent les cellules non désirées (lymphocytes B, monocytes,....). Les cellules sont centrifugées et ensuite lavées à deux reprises. Par analyse FACS on constate que les cultures de lymphocytes T ainsi produites contiennent plus de 85% de CD3 + cellules.

Ces cultures sont soumises à un traitement par diverses concentrations d'ADA. Les résultats sont donnés dans le tableau 3 ci-dessous.

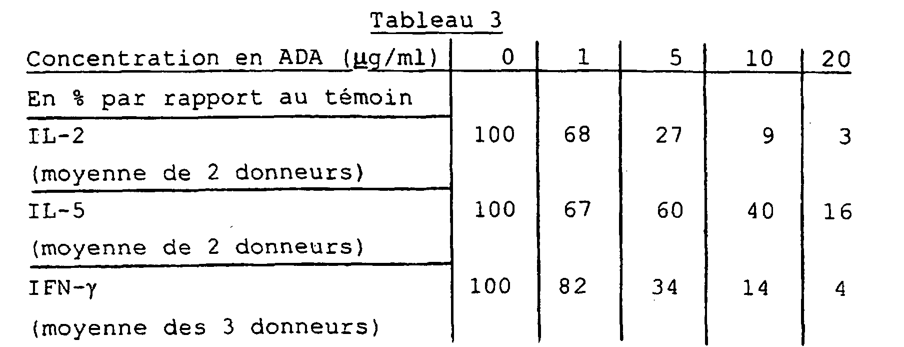

### Exemple 4

### Effet de l'ADA sur la production d'IL-2, d'IL-5 et d'interféron-γ par des lymphocytes T CD4 purifiés.

Des lymphocytes T CD4 purifiés (90% de pureté) et stimulés par du PMA (phorbol myristate acétate) + anticorps anti-CD28 de donneurs sains sont soumis à un traitement par diverses concentrations d'ADA. Les résultats sont donnés dans le tableau 4 ci-dessous.

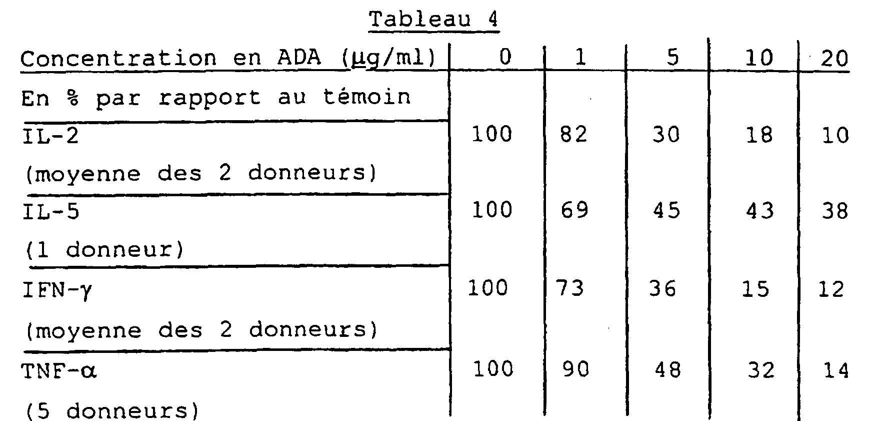

Dans les exemples 3 et 4, les résultats sont exprimés en % en considérant le milieu sans ADA comme 100%. Les concentrations correspondant à 100% sont respectivement de 90.000 pg/ml de IL-2, 1100 pg/ml de IL-5, 1900 pg/ml de IFNγ et 2870 pg/ml de TNF-α. Les résultats sont la moyenne de mesures ELISA indépendantes.

On constate, pour ces donneurs, que la production d'IL-5, d'interféron-γ et de TNF-α est inhibée à la concentration de 20 µg/ml d'ADA. Des concentrations inférieures inhibent déjà la sécrétion d'IL-2 par les lymphocytes.

Il ressort de ces expériences que l'ADA agit sur la production et la sécrétion de cytokines à des concentrations d'une manière surprenante très largement inférieures à celles nécessaires pour que cette substance ait une action contre la prolifération des rétrovirus VIH dans des cellules et contre la prolifération de cellules de type cancéreux, ce qui permet d'envisager son emploi, sans problème de toxicité, dans un domaine thérapeutique dans lequel on ne pouvait pas s'attendre jusqu'à présent à son application.

### Exemple 5

### Effet de l'ADA sur la production de cytokines in vivo.

On effectue une injection i.p. unique d'azodicarbonamide (ADA) en une dose de 100 mg/kg de poids de corps ou du support correspondant sur des souris BALB/C (Harlan, Zeist, Pays-Bas), un jour avant l'inoculation i.p. de 25 µg de 145-2C11 mAb de hamster, un anti-souris CD₃ mAb qui induit une activation massive des cellules T polyclonales in vivo conduisant à une libération systémique de cytokines. Des sérums sont collectés 2, 4, 8 et 24 heures après le traitement anti-CD₃ et les niveaux de cytokines (IL-2, IL-4) sont déterminés par un test ELISA (Genzyme).

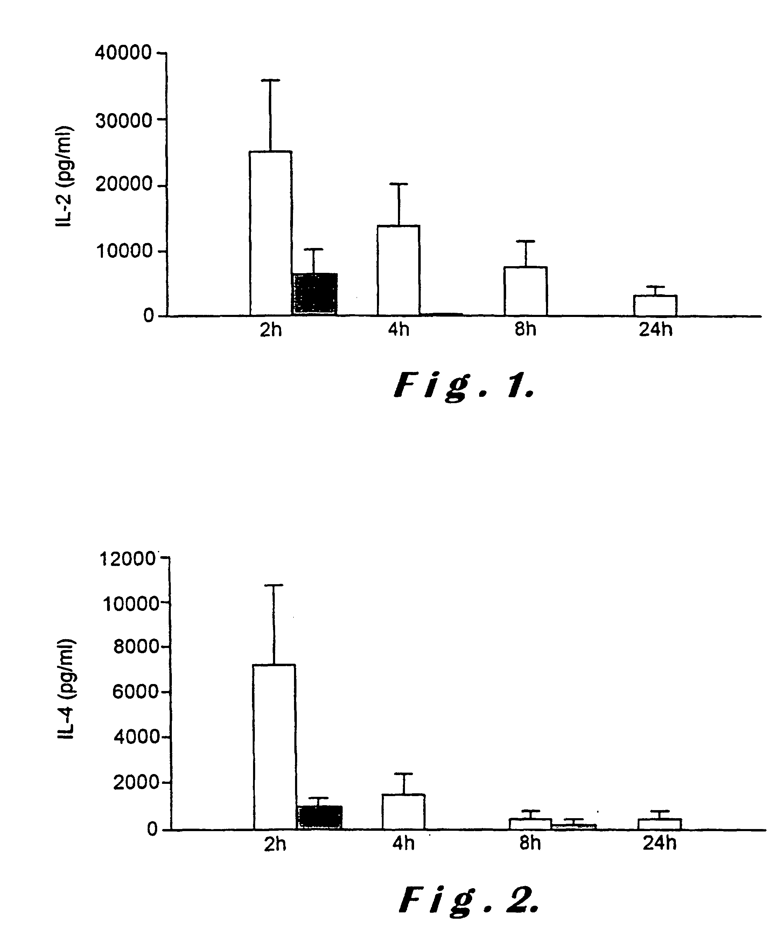

Sur les figures 1 et 2, on a représenté des graphiques avec en ordonnée les quantités de IL-2 et de IL-4 dans le sérum, sous la forme d'une moyenne de 5 animaux testés. En abscisse sont indiqués les moments des prélèvements de sang. Les barres vides correspondent aux résultats obtenus avec le support et les barres grisées à ceux obtenus avec un traitement préalable d'ADA.

Il ressort clairement de cet essai et des figures 1 et 2 qui l'illustrent que le traitement à l'ADA provoque une diminution significative de la libération des interleukines IL-2 et IL-4 après injection de 145-2C11 mAb dans des souris.

### Exemple 6

### Effet de l'ADA sur le rejet de greffes allogènes.

Des greffes de peau bien connues pour leur dépendance vis-à-vis des cellules T sont préparées à partir de queues de souris femelles C57BL/6.CH-2^{bm12} (Jackson Laboratory, Bar Harbor, ME, USA). Elles sont greffées sur les flancs de souris C57BL/6 (Harlan, Zeist, Pays-Bas). Un bandage est appliqué autour du trou. Ce bandage est enlevé 10 jours après et les greffes sont contrôlées chaque jour. Les souris d'essai reçoivent journellement une injection i.p. de 50 mg/kg de poids de corps d'ADA et les témoins reçoivent une quantité équivalente du support correspondant, jusqu'à ce qu'un rejet aigu soit observé.

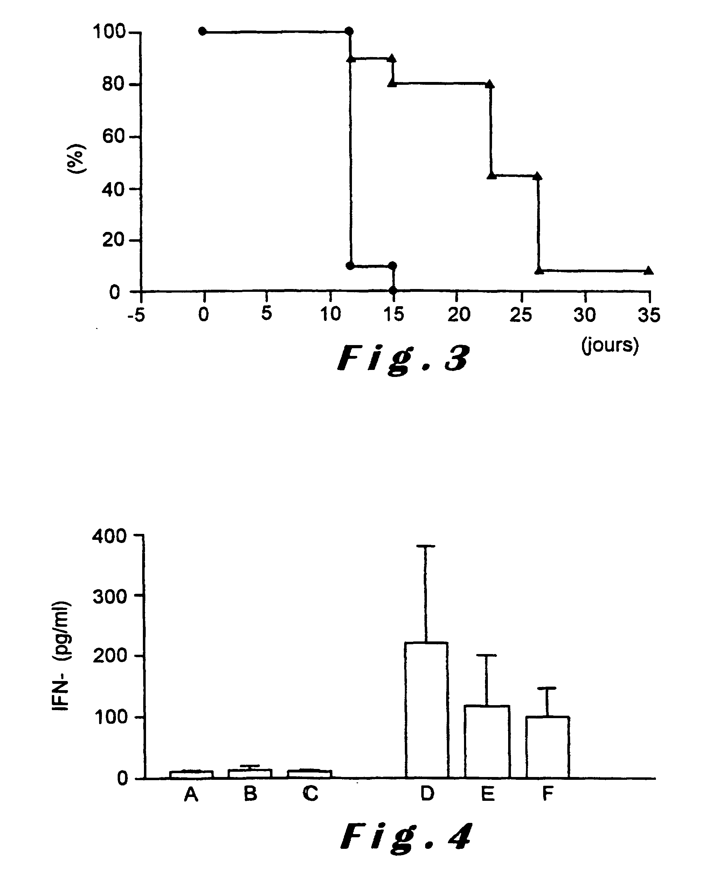

Sur le graphique de la figure 3 on a indiqué en ordonnée, en %, la survivance de la greffe de peau et en abscisse le nombre de jours après la transplantation.

On peut constater que le rejet de l'allogreffe de peau a été significativement retardé chez les souris traitées à l'ADA (triangles noirs) par rapport au témoin (cercles noirs), ce qui confirme que l'ADA exerce aussi des effets suppresseurs sur des cellules T in vivo.

### Exemple 7

200 ml de sang sont extraits sur l'héparine à partir de volontaires sains, mâles et femelles. Des cellules mononucléées sont isolées par centrifugation Ficoll-Hypaque et remises en suspension dans un milieu constitué de RPMI 1640 complété de 5% de sérum autologue, de 1 mN de glutamine, de 1001 IU/ml de pénicilline, de 10 µg/ml de streptomycine, de 10 µg/ml de phytohémagglutinine (pha), de 10 µg/ml d'acétate myristate de phorbol (pma) et de composés d'essai, comme indiqué, par expérience individuelle, à une densité cellulaire de 2.000.000 par ml. Les cellules sont ensemencées dans des plaques de microtitrage à 96 puits à 250 µl par puits et elles sont mises à incuber pendant 48 heures à 37°C. Ensuite, les surnageants sont récoltés et les concentrations en IL-2 et en IL-5 sont quantifiées par un ELISA standard, utilisant une technologie de biotine - streptavidine. En variante, la pha a été remplacée par 0,1 µg/ml d'anticorps monoclonaux dirigés contre des CD3 humains, de façon à obtenir une activation plus spécifique des cellules T.

**Tableau 5**

| | Inhibition de IL-2 (%) | | | Inhibition de IL-5 (%) | |
|---|---|---|---|---|---|
| Composés d'essai | Nombre d'essais | 10 µM | 32 µM | 10 µM | 32 µM |
| A | 9 | 3 | 28 | 12 | 29 |
| B | 6 | 0,2 | 11 | 17 | 39 |
| C | 5 | 6 | 9 | 37 | 51 |
| A = Diamide | | | | | |
| B = 1,1'-(azodicarbonyl)-dipipéridine | | | | | |
| C = 1,1'-(azodicarbonyl)-dimorpholine | | | | | |

### Exemple 8

### Effet de l'ADA sur la production in vivo d'interféron-γ.

Des rats Brown-Norway sont sensibilisés activement à l'ovalbumine 21 jours plus tard, les rats sont traités par voie d'aérosol avec une solution d'ovalbumine à 5% dans du NaCl 0,9%. 24 heures plus tard, la réactivité bronchique à la métacholine est mesurée sur les rats anesthésiés. Dès la fin de la mesure de réactivité bronchique, des splénocytes sont prélevés de ces rats et mis en culture pour déterminer la production d'IFN-γ par ces cellules.

De l'ADA est administré 24 heures avant le début de la phase de sensibilisation à l'ovalbumine et quotidiennement pendant toute la durée de l'essai, à l'exception du jour des mesures. L'ADA est administré par voie orale, en suspension dans de la méthylcellulose 1%, en un dosage de 500 mg/kg et de 1000 mg/kg de poids corps, deux fois par jour.

Les résultats sont réunis sur la figure 4. En ordonnée sont indiquées les quantités d'IFN-γ en pg/mg de protéine. En abscisse les barres vides A à F correspondent aux expérimentations suivantes :
A - pas de stimulation à l'ovalbumine, pas de substance active
B - pas de stimulation à l'ovalbumine, ADA à 500 mg/kg p.o.
C - pas de stimulation à l'ovalbumine, ADA à 1000 mg/kg p.o.
D - stimulation à l'ovalbumine, pas de substance active
E - stimulation à l'ovalbumine, ADA à 500 mg/kg p.o.
F - stimulation à l'ovalbumine, ADA à 1000 mg/kg p.o.

Il ressort clairement de cet essai que l'ADA a un effet d'inhibition de la production d'INF-γ par les splénocytes de rats traités pour montrer une forte réactivité bronchique.

### Exemple 9

### Gélules à administrer par voie orale.

Composition d'une gélule :
500 mg d'ADA
10 mg de monostéarate de glycérine
10 mg de dioxyde de silicium précipité
5 mg de stéarate de magnésium.

On introduit d'une manière courante cette composition dans des capsules de gélatine. On peut par exemple prévoir une administration de gélules à dose croissante (maximum 100 mg/kg/jour) à des patients qui reçoivent une greffe rénale ou autre allogreffe (coeur, poumon, foie, etc...) dans les 72 heures au plus tard précédant la transplantation, afin de diminuer ou de supprimer la phase de rejet aiguë. Un contrôle fréquent de la production d'IL-2 par les patients est recommandé. Les gélules seront administrées seules ou en association avec de la cyclosporine et des corticostéroïdes ou tout autre traitement immunosuppresseur approprié.

### Exemple 10

### Tablettes enrobées.

On réalise d'une manière courante des tablettes contenant 250 mg d'azobisformamidine à l'aide des excipients suivants : hydroxypropylméthylcellulose, hydroxypropylcellulose, dioxyde de titane, polyéthylèneglycol 400, oxyde noir de fer.

Ces tablettes peuvent être administrées pour réduire de manière adéquate la production d'IgE grâce à la diminution d'IL-5 dans les phénomènes allergiques (rhinite allergique saisonnière par exemple). Ce traitement sera instauré afin d'obtenir une rémission et son maintien.

### Exemple 11

### Forme à injecter.

On réalise une forme injectable à base de 1g de diméthylazobisformamide et d'eau distillée apyrogène additionnée de NaCl.

Ces formes sont à administrer par exemple dans les phases de poussées aiguës de production d'autoanticorps (maladies autoimmunes, lupus erythémateux, diabète autoimmun, thyroïdite autoimmune, etc...) tout en gardant à l'esprit que l'amélioration s'observe habituellement après un délai de six à huit semaines.

### Exemple 12

### Crème ou onguent.

Une crème ou un onguent est réalisé avec de la 1,1'-azobis-(chloroformamidine] (choroazodine) et comme excipient, notamment de la glycérine, de l'huile de paraffine, de la vaseline.

Cette crème peut être appliquée localement à titre d'immunomodulateur dans la sclérodermie.

### Exemple 13

On peut aussi prévoir des systèmes de distribution transdermique de diméthylazobisformamide.

Ces systèmes peuvent être appliqués de manière à obtenir une diminution soutenue des lymphokines circulant chez des patients souffrant d'asthme difficile à contrôler.

Ce traitement peut être combiné avec l'usage de traitements conventionnels.

### Exemple 14

### Comprimés à administrer par voie orale.

Composition d'un comprimé :
100 mg d'ADA
10 mg de monostéarate de glycérine
10 mg de dioxyde de silicium précipité
5 mg de stéarate de magnésium.

On introduit d'une manière courante cette composition dans une machine à comprimer.

Dans le cadre d'un traitement par exemple d'un cancer, on peut prévoir une administration d'IL-2 au patient. La teneur surabondante d'IL-2 dans le corps du patient ainsi traité a pour effet de stimuler une surproduction par les cellules de ce dernier d'autres cytokines, et notamment d'IL-5, ce qui entraîne des effets secondaires, tels que pemphigus, thyroïdite, polyarthrite rhumatoïde, maladie de Crohn, sclérodermie, hyperéosinophilie, etc....

On peut par conséquent prévoir conjointement à l'administration d'IL-2, la prise régulière de comprimés d'ADA pour contrecarrer ces effets secondaires.

L'administration d'IL-2 et d'ADA peut se faire simultanément, séparément ou d'une manière étalée dans le temps.

## Revendications

1. Procédé d'inhibition in vitro de production de cytokines par des cellules, notamment animales ou humaines, et de leur sécrétion, comprenant une application sur ces cellules d'au moins un des dérivés azoïques répondant à la formule 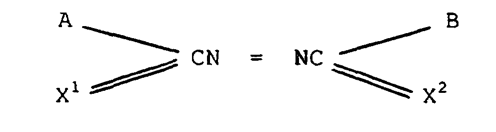 dans laquelle A représente un groupe carboxyle ou un groupe 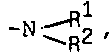 B représente un groupe carboxyle ou un groupe 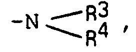 R¹, R², R³ et R⁴ sont identiques ou différents et représentent chacun indépendamment un atome d'hydrogène ou d'halogène, un radical d'hydrocarbure aliphatique ou aromatique, éventuellement substitué, ou un groupe hydroxy, R¹ et R² pouvant être liés ensemble pour former un noyau hétérocyclique avec leur atome d'azote adjacent, et R³ et R⁴ pouvant être liés ensemble pour former un noyau hétérocyclique avec leur atome d'azote adjacent, X¹ et X² sont identiques ou différents et représentent chacun indépendamment un atome d'oxygène ou un groupe NR⁵, dans lequel R⁵ est un atome d'hydrogène ou d'halogène, un radical d'hydrocarbure aliphatique ou aromatique, éventuellement substitué, ou un groupe nitro, et dans lequel, lorsque deux groupes NR⁵ sont simultanément présents, chaque R⁵ peut être identique à ou différent de l'autre, ainsi que de leurs sels, esters et isomères.

2. Procédé suivant la revendication 1, **caractérisé en ce qu'**il comprend une inhibition de production et de sécrétion d'interleukines.

3. Procédé suivant la revendication 1, **caractérisé en ce qu'**il comprend une inhibition de production et de sécrétion d'une cytokine choisie parmi le groupe comprenant de l'interféron-γ et du facteur de nécrose tumorale α.

4. Procédé suivant l'une quelconque des revendications 1 à 3, **caractérisé en ce que** R₁ à R₅ représentent chacun un radical d'hydrocarbure aliphatique ou aromatique comportant de 1 à 6 atomes de carbone.

5. Procédé suivant l'une quelconque des revendications 1 à 4, **caractérisé en ce que** le dérivé azoïque est choisi parmi le groupe comprenant des dérivés d'azobisformamidine, tels que de la 1,1'-azobisformamidine, de la 1,1'-azobisnitroformamidine, de la 2,2'-azobisméthylformamidine, de la 1,1'-azobisfluoroformamidine, de la 1-monochloroazobisformamidine, et de la azobis-[chloroformamidine], des dérivés d'azobisformamide, tels que du 1,1'-azobisformamide et du diméthyl-azobisformamide, de la 1,1'-(azodicarbonyl)-dipipéridine, de la 1,1'-(azodicarbonyl)-dimorpholine, de l'acide azodihydroxamique et ses sels, et de l'acide azodicarboxylique et ses sels.

6. Procédé suivant l'une quelconque des revendications 1 à 5, **caractérisé en ce qu'**il comprend une application du dérivé azoïque sur les cellules à une dose n'induisant pas une apoptose de celles-ci.

7. Procédé suivant l'une quelconque des revendications 1 à 6, **caractérisé en ce qu'**il comprend une application du dérivé azoïque sur les cellules à une concentration micromolaire de 0,4 à 200.

8. Procédé suivant la revendication 7, **caractérisé en ce qu'**il comprend une application du dérivé azoïque sur les cellules à une concentration micromolaire de 2 à 20, de préférence de 2 à 10.

9. Procédé suivant l'une quelconque des revendications 1 à 8, **caractérisé en ce qu'**il comprend l'application susdite d'une composition comportant au moins un desdits dérivés azoïques et un support approprié.

10. Procédé suivant l'une quelconque des revendications 1 à 9, **caractérisé en ce qu'**il comprend ladite application sur des cellules isolées de macroorganismes ou des cellules de microorganismes.

11. Procédé suivant l'une quelconque des revendications 1 à 9, **caractérisé en ce qu'**il comprend ladite application sur des cellules d'un organe ou tissu multicellulaire extrait d'un corps humain ou animal.

12. Utilisation d'au moins un des dérivés azoïques répondant à la formule 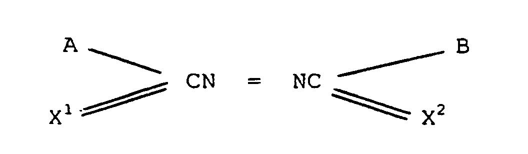 dans laquelle A représente un groupe carboxyle ou un groupe 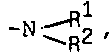 B représente un groupe carboxyle ou un groupe 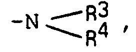 R¹, R², R³ et R⁴ sont identiques ou différents et représentent chacun indépendamment un atome d'hydrogène ou d'halogène, un radical d'hydrocarbure aliphatique ou aromatique, éventuellement substitué, ou un groupe hydroxy, R¹ et R² pouvant être liés ensemble pour former un noyau hétérocyclique avec leur atome d'azote adjacent, et R³ et R⁴ pouvant être liés ensemble pour former un noyau hétérocyclique avec leur atome d'azote adjacent, X¹ et X² sont identiques ou différents et représentent chacun indépendamment un atome d'oxygène ou un groupe NR⁵, dans lequel R⁵ est un atome d'hydrogène ou d'halogène, un radical d'hydrocarbure aliphatique ou aromatique, éventuellement substitué, ou un groupe nitro, et dans lequel, lorsque deux groupes NR⁵ sont simultanément présents, chaque R⁵ peut être identique à ou différent de l'autre, ainsi que de leurs sels, esters et isomères, pour la fabrication de médicaments à mettre en oeuvre dans le traitement ou la prophylaxie d'affections humaines ou animales résultant d'une production cellulaire pathologique de cytokines, à l'exception des maladies virales.

13. Utilisation suivant la revendication 12, **caractérisée en ce que** le médicament ainsi fabriqué contient une quantité thérapeutiquement efficace d'au moins un desdits dérivés azoïques ou de leurs sels, esters et isomères ainsi qu'un support pharmaceutiquement approprié.

14. Dérivés azoïques répondant à la formule de la revendication 1, dans laquelle au moins un des groupes A et B représente un groupe carboxyle, ainsi que leurs sels, esters et isomères pharmaceutiquement acceptables, pour leur application en tant que substances thérapeutiquement actives, à mettre en oeuvre dans le traitement ou la prophylaxie d'affections humaines ou animales résultant d'une production cellulaire pathologique de cytokines.

15. Dérivés azoïques répondant à la formule de la revendication 1, dans laquelle le groupe A représente le groupe 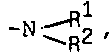 l'un des radicaux R¹ et R² représentant un groupe hydroxy et l'autre un atome d'hydrogène, et dans laquelle le groupe B peut représenter simultanément le groupe 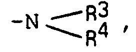 l'un des radicaux R³ et R⁴ pouvant représenter un groupe hydroxy et l'autre un atome d'hydrogène, ainsi que leurs sels, esters et isomères pharmaceutiquement acceptables, pour leur application en tant que substances thérapeutiquement actives, à mettre en oeuvre dans le traitement ou la prophylaxie d'affections humaines ou animales résultant d'une production cellulaire pathologique de cytokines.

16. Dérivés azoïques répondant à la formule de la revendication 1, dans laquelle X¹ et X² représentent de l'oxygène, et NR¹R² ainsi que NR³R⁴ représentent chacun un hétérocycle hexagonal contenant en supplément un atome d'oxygène en position 4, ainsi que leurs sels, esters et isomères pharmaceutiquement acceptables, pour leur application en tant que substances thérapeutiquement actives, à mettre en oeuvre dans le traitement ou la prophylaxie d'affections humaines ou animales résultant d'une production cellulaire pathologique de cytokines.

17. Produit contenant au moins un des dérivés azoïques répondant à la formule 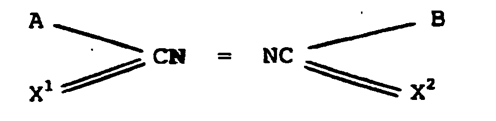 dans laquelle A représente un groupe carboxyle ou un groupe 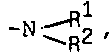 B représente un groupe carboxyle ou un groupe 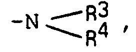 R¹, R², R³ et R⁴ sont identiques ou différents et représentent chacun indépendamment un atome d'hydrogène ou d'halogène, un radical d'hydrocarbure aliphatique ou aromatique, éventuellement substitué, ou un groupe hydroxy, R¹ et R² pouvant être liés ensemble pour former un noyau hétérocyclique avec leur atome d'azote adjacent, et R³ et R⁴ pouvant être liés ensemble pour former un noyau hétérocyclique avec leur atome d'azote adjacent, X¹ et X² sont identiques ou différents et représentent chacun indépendamment un atome d'oxygène ou un groupe NR⁵, dans lequel R⁵ est un atome d'hydrogène ou d'halogène, un radical d'hydrocarbure aliphatique ou aromatique, éventuellement substitué, ou un groupe nitro, et dans lequel, lorsque deux groupes NR⁵ sont simultanément présents, chaque R⁵ peut être identique à ou différent de l'autre, ainsi que leurs sels, esters ou isomères, **caractérisé en ce qu'**il comprend en outre au moins une cytokine, comme produit de combinaison pour une utilisation simultanée, séparée ou étalée dans le temps, dans la thérapie ou la prophylaxie d'affections humaines ou animales résultant d'une production cellulaire pathologique d'au moins une cytokine, différente de ladite au moins une cytokine contenue dans le produit de combinaison, ou dans la thérapie ou la prophylaxie d'affections humaines ou animales ayant pour effet secondaire une production cellulaire pathologique d'au moins une cytokine, différente de ladite au moins une cytokine contenue dans le produit de combinaison.

## Patentansprüche

1. Verfahren zur In-vitro-Inhibierung der Produktion von Cytokinen durch Zellen, insbesondere tierische oder menschliche, und ihrer Sekretion, umfassend eine Applikation mindestens eines der Azoderivate der Formel 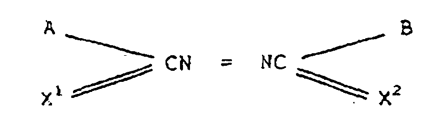 wobei A eine Carboxylgruppe oder einen Rest -NR¹R² darstellt, B eine Carboxylgruppe oder einen Rest -NR³R⁴ darstellt, R¹, R², R³, und R⁴ gleich oder verschieden sind und jeweils unabhängig ein Wasserstoff- oder Halogenatom, einen gegebenenfalls substituierten aliphatischen oder aromatischen Kohlenwasserstoffrest oder eine Hydroxygruppe darstellen, R¹ und R² miteinander verbunden sein können, um mit ihrem benachbarten Stickstoffatom einen heterocyclischen Ring zu bilden, und R³ und R⁴ miteinander verbunden sein können, um mit ihrem benachbarten Stickstoffatom einen heterocyclischen Ring zu bilden, X¹ und X² gleich oder verschieden sind und jeweils unabhängig ein Sauerstoffatom oder einen Rest NR⁵ darstellen, wobei R⁵ ein Wasserstoff- oder Halogenatom, einen gegebenenfalls substituierten aliphatischen oder aromatischen Kohlenwasserstoffrest oder eine Nitrogruppe darstellt und wobei, wenn zwei Reste NR⁵ gleichzeitig vorhanden sind, jeder Rest R⁵ gleich oder verschieden vom anderen sein kann, sowie ihrer Salze, Ester und Isomere auf diese Zellen.

2. Verfahren gemäß Anspruch 1, **dadurch gekennzeichnet, dass** es eine Inhibierung der Produktion und der Sekretion von Interleukinen umfasst.

3. Verfahren gemäß Anspruch 1, **dadurch gekennzeichnet, dass** eine Inhibierung der Produktion und der Sekretion eines Cytokins, ausgewählt aus γ-Interferon und dem Tumor-Nekrose-Faktor α, umfasst.

4. Verfahren gemäß einem der Ansprüche 1 bis 3, **dadurch gekennzeichnet, dass** R¹ bis R⁵ jeweils einen aliphatischen oder aromatischen Kohlenwasserstoffrest mit 1 bis 6 Kohlenstoffatomen darstellen.

5. Verfahren gemäß einem der Ansprüche 1 bis 4, **dadurch gekennzeichnet, dass** das Azoderivat aus Azobisformamidinderivaten wie 1,1'-Azobisformamidin, 1,1'-Azobisnitroformamidin, 2,2'-Azobismethylformamidin, 1,1'-Azobisfluorformamidin, 1-Monochlorazobisformamidin und Azobis(chlorformamidin), Azobisformamidderivaten wie 1,1'-Azobisformamid und Dimethylazobisformamid, 1,1'-(Azodicarbonyl)dipiperidin, 1,1'-(Azodicarbonyl)dimorpholin, Azodihydroxamsäure und ihren Salzen und Azodicarbonsäure und ihren Salzen, ausgewählt ist.

6. Verfahren gemäß einem der Ansprüche 1 bis 5, **dadurch gekennzeichnet, dass** es eine Applikation des Azoderivats auf die Zellen in einer Dosis umfasst, die nicht deren Apoptose induziert.

7. Verfahren gemäß einem der Ansprüche 1 bis 6, **dadurch gekennzeichnet, dass** es eine Applikation des Azoderivats auf die Zellen in einer Konzentration von 0,4 bis 200 Mikromol umfasst.

8. Verfahren gemäß Anspruch 7, **dadurch gekennzeichnet, dass** es eine Applikation des Azoderivats auf die Zellen in einer Konzentration von 2 bis 20 Mikromol, vorzugsweise von 2 bis 10 Mikromol, umfasst.

9. Verfahren gemäß einem der Ansprüche 1 bis 8, **dadurch gekennzeichnet, dass** es die Applikation einer Zusammensetzung, enthaltend mindestens eines der Azoderivate und einen geeigneten Träger, umfasst.

10. Verfahren gemäß einem der Ansprüche 1 bis 9, **dadurch gekennzeichnet, dass** es die Applikation auf isolierte Zellen von Mikroorganismen oder Zellen von Mikroorganismen umfasst.

11. Verfahren gemäß einem der Ansprüche 1 bis 9, **dadurch gekennzeichnet, dass** es die Applikation auf Zellen eines Organs oder multizellulären Gewebes, das aus einem menschlichen oder tierischen Körper entnommen ist, umfasst.

12. Verwendung mindestens eines der Azoderivate der Formel 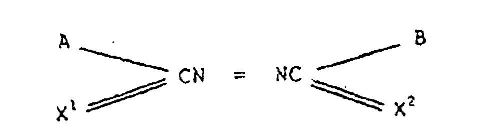 wobei A eine Carboxylgruppe oder einen Rest -NR¹R² darstellt, B eine Carboxylgruppe oder einen Rest -NR³R⁴ darstellt, R¹, R², R³ und R⁴ gleich oder verschieden sind und jeweils unabhängig ein Wasserstoff- oder Halogenatom, einen gegebenenfalls substituierten aliphatischen oder aromatischen Kohlenwasserstoffrest oder eine Hydroxygruppe darstellen, R¹ und R² miteinander verbunden sein können, um mit ihrem benachbarten Stickstoffatom einen heterocyclischen Ring zu bilden, und R³ und R⁴ miteinander verbunden sein können, um mit ihrem benachbarten Stickstoffatom einen heterocyclischen Ring zu bilden, X¹ und X² gleich oder verschieden sind und jeweils unabhängig ein Sauerstoffatom oder einen Rest NR⁵ darstellen, wobei R⁵ ein Wasserstoff- oder Halogenatom, einen gegebenenfalls substituierten aliphatischen oder aromatischen Kohlenwasserstoffrest oder eine Nitrogruppe darstellt und wobei, wenn zwei Reste NR⁵ gleichzeitig vorhanden sind, jeder Rest R⁵ gleich oder verschieden vom anderen sein kann, sowie ihrer Salze, Ester und Isomere, zur Herstellung von Medikamenten zum Einsatz in der Behandlung oder der Prophylaxe von menschlichen oder tierischen Erkrankungen infolge einer pathologischen zellulären Produktion von Cytokinen, mit Ausnahme viraler Erkrankungen.

13. Verwendung gemäß Anspruch 12, **dadurch gekennzeichnet, dass** das so hergestellte Medikament eine therapeutisch wirksame Menge mindestens eines der Azoderivate oder ihrer Salze, Ester und Isomere sowie einen pharmazeutisch verträglichen Träger enthält.

14. Azoderivate der Formel des Anspruchs 1, wobei mindestens einer der Reste A und B eine Carboxylgruppe darstellt, sowie ihre pharmazeutisch verträglichen Salze, Ester und Isomere zur Applikation als therapeutische Wirkstoffe zum Einsatz in der Behandlung oder der Prophylaxe von menschlichen oder tierischen Erkrankungen infolge einer pathologischen zellulären Produktion von Cytokinen.

15. Azoderivate der Formel des Anspruchs 1, wobei der Rest A den Rest -NR¹R² darstellt, einer der Reste R¹ und R² eine Hydroxygruppe und der andere ein Wasserstoffatom darstellt und wobei der Rest B gleichzeitig den Rest -NR³R⁴ darstellen kann, einer der Reste R³ und R⁴ eine Hydroxygruppe und der andere ein Wasserstoffatom darstellen kann. sowie ihre pharmazeutisch verträglichen Salze, Ester und Isomere zur Applikation als therapeutische Wirkstoffe zum Einsatz in der Behandlung oder der Prophylaxe von menschlichen oder tierischen Erkrankungen infolge einer pathologischen zellulären Produktion von Cytokinen.

16. Azoderivate der Formel des Anspruchs 1, wobei X¹ und X² ein Sauerstoffatom darstellen und -NR¹R² sowie -NR³R⁴ jeweils einen 6-gliedrigen Heterocyclus, der zusätzlich ein Sauerstoffatom in Position 4 enthält, darstellen, sowie ihre pharmazeutisch verträglichen Salze, Ester und Isomere, zur Applikation als therapeutische Wirkstoffe zum Einsatz in der Behandlung oder der Prophylaxe von menschlichen oder tierischen Erkrankungen infolge einer pathologischen zellulären Produktion von Cytokinen.

17. Präparat, enthaltend mindestens eines der Azoderivate der Formel 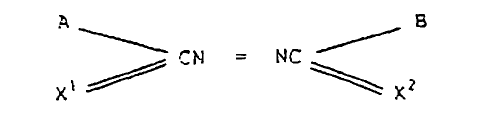 wobei A eine Carboxylgruppe oder einen Rest -NR¹R² darstellt, B eine Carboxylgruppe oder einen Rest -NR³R⁴ darstellt, R¹, R², R³ und R⁴ gleich oder verschieden sind und jeweils unabhängig ein Wasserstoff- oder Halogenatom, einen gegebenenfalls substituierten aliphatischen oder aromatischen Kohlenwasserstoffrest oder eine Hydroxygruppe darstellen, R¹ und R² miteinander verbunden sein können, um mit ihrem benachbarten Stickstoffatom einen heterocyclischen Ring zu bilden, und R³ und R⁴ miteinander verbunden sein können, um mit ihrem benachbarten Stickstoffatom einen heterocyclischen Ring zu bilden, X¹ und X² gleich oder verschieden sind und jeweils unabhängig ein Sauerstoffatom oder einen Rest NR⁵ darstellen, wobei R⁵ ein Wasserstoff- oder Halogenatom, einen gegebenenfalls substituierten aliphatischen oder aromatischen Kohlenwasserstoffrest oder eine Nitrogruppe darstellt und wobei, wenn zwei Reste NR⁵ gleichzeitig vorhanden sind, jeder Rest R⁵ gleich oder verschieden vom anderen sein kann, sowie ihre Salze, Ester oder Isomere, **dadurch gekennzeichnet, dass** es zudem mindestens ein Cytokin umfasst, als Kombinationspräparat für eine gleichzeitige, getrennte oder zeitlich versetzte Verwendung in der Therapie oder der Prophylaxe von menschlichen oder tierischen Erkrankungen infolge einer pathologischen zellulären Produktion mindestens eines Cytokins. das sich von dem mindestens einen, in dem Kombinationspräparat enthaltenen Cytokin unterscheidet, oder in der Therapie oder der Prophylaxe von menschlichen oder tierischen Erkrankungen, die als Nebeneffekt eine pathologische zelluläre Produktion mindestens eines Cytokins, das sich von dem mindestens einen in dem Kombinationspräparat enthaltenen Cytokin unterscheidet, aufweisen.

## Claims

1. Process for *in vitro* inhibition of the production of cytokines by cells, in particular animal or human cells, and of the secretion thereof, comprising application onto said cells of at least one azo derivative of the formula 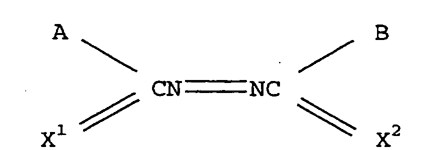 in which A represents a carboxyl group or a group 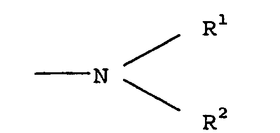 B represents a carboxyl group or a group 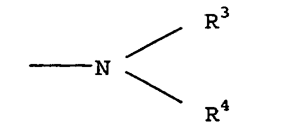 R¹, R², R³ and R⁴ are identical or different and each mutually independently represent a hydrogen or halogen atom, an optionally substituted aliphatic or aromatic hydrocarbon residue or a hydroxy group, R¹ and R² possibly being joined together to form a heterocyclic nucleus with the nitrogen atom adjacent thereto, and R³ and R⁴ possibly being joined together to form a heterocyclic nucleus with the nitrogen atom adjacent thereto, X¹ and X² are identical or different and each mutually independently represent an oxygen atom or a group NR⁵, in which R⁵ is a hydrogen or halogen atom, an optionally substituted aliphatic or aromatic hydrocarbon residue or a nitro group, and in which, when two groups NR⁵ are simultaneously present, each R⁵ may be identical to or different from the other, as well as the salts, esters and isomers thereof.

2. Process according to claim 1, **characterised in that** it comprises inhibition of the production and secretion of interleukins.

3. Process according to claim 1, **characterised in that** it comprises inhibition of the production and secretion of a cytokine selected from the group comprising interferon γ and tumour necrosis factor α.

4. Process according to any of claims 1 to 3, **characterised in that** R¹ to R⁵ each represent an aliphatic or aromatic hydrocarbon residue comprising 1 to 6 carbon atoms.

5. Process according to any of claims 1 to 4, **characterised in that** the azo derivative is selected from the group comprising azobisformamidine derivatives, such as 1,1'-azobisformamidine, 1,1'-azobisnitroformamidine, 2,2'-azobismethylformamidine, 1,1'-azobisfluoroformamidine, 1-monochloroazobisformamidine, azobis[chloroformamidine], azobisformamide derivatives, such as 1,1'-azobisformamide and dimethylazobisformamide, 1,1'-(azodicarbonyl)dipiperidine, 1,1'-(azodicarbonyl)-dimorpholine, azodihydroxamic acid and the salts thereof, azodicarboxylic acid and the salts thereof.

6. Process according to any of claims 1 to 5, **characterised in that** it comprises application of the azo derivative onto cells at a dose which does not induce apoptosis thereof.

7. Process according to any of claims 1 to 6, **characterised in that** it comprises application of the azo derivative onto the cells at a micromolar concentration of 0.4 to 200.

8. Process according to claim 7, **characterised in that** it comprises application of the azo derivative onto the cells at a micromolar concentration of 2 to 20, preferably of 2 to 10.

9. Process according to any of claims 1 to 8, **characterised in that** it comprises said application of a composition comprising at least one of said azo derivatives and an appropriate carrier.

10. Process according to any of claims 1 to 9, **characterised in that** it comprises said application onto cells isolated from macroorganisms or cells of microorganisms.

11. Process according to any of claims 1 to 9, **characterised in that** it comprises said application onto cells from an organ or multicellular tissue taken from the body of a human or an animal.

12. Use of at least one of the azo derivatives of the formula 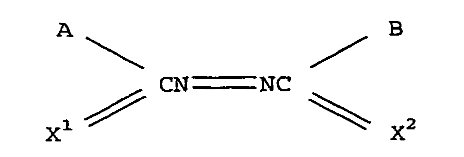 in which A represents a carboxyl group or a group 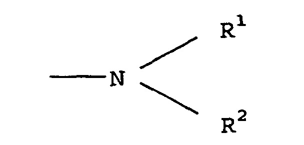 B represents a carboxyl group or a group 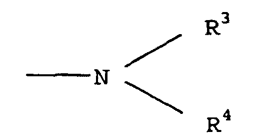 R¹, R², R³ and R⁴ are identical or different and each mutually independently represent a hydrogen or halogen atom, an optionally substituted aliphatic or aromatic hydrocarbon residue or a hydroxy group, R¹ and R² possibly being joined together to form a heterocyclic nucleus with the nitrogen atom adjacent thereto, and R³ and R⁴ possibly being joined together to form a heterocyclic nucleus with the nitrogen atom adjacent thereto, X¹ and X² are identical or different and each mutually independently represent an oxygen atom or a group NR⁵, in which R⁵ is a hydrogen or halogen atom, an optionally substituted aliphatic or aromatic hydrocarbon residue or a nitro group, and in which, when two groups NR⁵ are simultaneously present, each R⁵ may be identical to or different from the other, together with the salts, esters and isomers thereof, for manufacturing medicines to be used in the treatment or prevention of human or animal conditions arising from pathological cellular production of cytokines, with the exception of viral diseases.

13. Use according to claim 12, **characterised in that** the medicine produced in this manner contains a therapeutically effective quantity of at least one of said azo derivatives or the salts, esters and isomers thereof as well as a pharmaceutically appropriate carrier.

14. Azo derivatives of the formula of claim 1 in which at least one of the groups A and B represents a carboxyl group, as well as the pharmaceutically acceptable salts, esters and isomers thereof, for application thereof as therapeutically active substances to be used in the treatment or prevention of human or animal conditions arising from pathological cellular production of cytoki nes.

15. Azo derivatives of the formula of claim 1, in which the groupe A represents the group 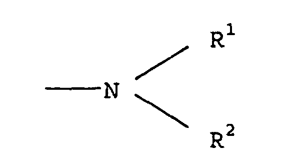 one of the residues R¹ and R² representing a hydroxy group and the other a hydrogen atom, and in which the group B may simultaneously represent the group 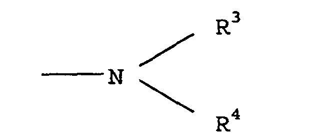 one of the residues R³ and R⁴ possibly representing a hydroxy group and the other a hydrogen atom, as well as the pharmaceutically acceptable salts, esters and isomers thereof, for the application thereof as therapeutically active substances to be used in the treatment or prevention of human or animal conditions arising from pathological cellular production of cytokines.

16. Azo derivatives of the formula of claim 1, in which X¹ and X² represent oxygen, and NR¹R² and NR³R⁴ each represent a hexagonal heterocycle additionally containing an oxygen atom in position 4, as well as the pharmaceutically acceptable salts, esters and isomers thereof, for the application thereof as therapeutically active substances to be used in the treatment or prevention of human or animal conditions arising from pathological cellular production of cytokines.

17. Product containing at least one of the azo derivatives of the formula 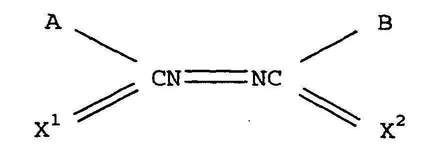 in which A represents a carboxyl group or a group 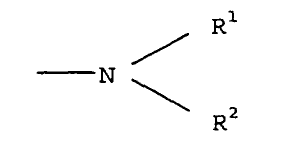 B represents a carboxyl group or a group 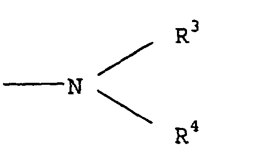 R¹, R², R³ and R⁴ are identical or different and each mutually independently represent a hydrogen or halogen atom, an optionally substituted aliphatic or aromatic hydrocarbon residue or a hydroxy group, R¹ and R² possibly being joined together to form a heterocyclic nucleus with the nitrogen atom adjacent thereto, and R³ and R⁴ possibly being joined together to form a heterocyclic nucleus with the nitrogen atom adjacent thereto, X¹ and X² are identical or different and each mutually independently represent an oxygen atom or a group NR⁵, in which R⁵ is a hydrogen or halogen atom, an optionally substituted aliphatic or aromatic hydrocarbon residue or a nitro group, and in which, when two groups NR⁵ are simultaneously present, each R⁵ may be identical to or different from the other as well as the salts, esters and isomers thereof, **characterised in that** it additionally comprises at least one cytokine as a combination product for use simultaneously, separately or in a staggered manner in the therapy or prevention of human or animal conditions arising from pathological cellular production of at least one cytokine differing from said at least one cytokine, which is present in the combination product, or in the therapy or prevention of human conditions, a side effect of which is pathological cellular production of at least one cytokine differing from said at least one cytokine, which is present in the combination product.
